# EUROPEAN PATENT APPLICATION

(11) **EP 2 072 621 A1**
(43) Date of publication of application: **24.06.2009**
(21) Application number: 07123947.9
(22) Date of filing: 21.12.2007
(51) Int. Cl.: C12P 7/16, C12N 1/20

(54) **Solvent tolerant microorganisms**

(71) Applicant: DSM IP Assets B.V., 6411 TE Heerlen (NL)
(72) Inventor: Smoor, Truke Widel, 2518 JJ, DEN HAAG (NL); Laat De, Wilhelmus Theodorus Antonius Maria, 4817 KW, BREDA (NL); Mueller, Ulrike Maria, 52441, Linnich (DE)
(74) Representative: Cazemier, Anne Engeline

(57) **Abstract**

The present invention relates to the use of a solvent tolerant lactic acid bacterium in a method for the fermentative production of a solvent. In particular, it relates to the use of solvent tolerant bacterium which belongs to the genera *Lactobacillus, Oenococcus* or *Leuconostoc*.in the production of solvent. These bacteria are particularly useful for this purpose because of their high IC50 (%) values for solvent and because they are well-established industrial organisms, which are widely used for industrial scale fermentations, in particular in the food industry, have GRAS status (generally recognized as safe) as fermentation agents in food and are not associated with health risks.

## Description

### Field of the invention

The present invention relates to solvent tolerant microorganisms, in particular to butanol tolerant microorganisms.

### Background of the invention

The acetone/butanol/ethanol (ABE) fermentation process has received considerable attention in the recent years as a prospective process for the production of commodity chemicals, such as butanol and acetone from biomass.

The fermentation of carbohydrates to acetone, butanol, and ethanol by solventogenic *Clostridia* is well known since decades. *Clostridia* produce butanol by conversion of a suitable carbon source into acetyl-CoA. Substrate acetyl-CoA then enters into the solventogenesis pathway to produce butanol using six concerted enzyme reactions.

The formation of butanol requires the conversion of acetyl-CoA into acetoacetyl-CoA by acetyl transferase. This reaction is followed by the conversion of acetoacetyl-CoA into 3-hydroxylbutyryl-CoA by 3-hydroxyl-CoA dehydrogenase, which is followed by the conversion of 3-hydroxylbutyryl-CoA into crotonyl-CoA by 3-hydroxybutyryl-CoA dehydratase (also named crotonase) and the conversion of crotonyl-CoA into butyryl-CoA by butyryl-CoA dehydrogenase and followed by the conversion of butyryl-CoA to butyraldehyde by butyraldehyde dehydrogenase, with the final conversion of butyrylaldehyde to butanol by butanol dehydrogenase (Jones, D.T., Woods, D.R., 1986, Microbiol. Rev., 50, 484-524).

However, the production of butanol suffers from poor process economics, because the butanol produced is toxic for the microbial cells and thus titers are low. Many studies have been directed to increase the resistance of *Clostridia* strains against butanol and consequently achieve an increase in titers. In US 6,960,465, it is for instance shown that overexpression of the heat shock proteins in *Clostridium acetobutylicum* resulted in an increased butanol production yield compared to the wild type strain.

Despite the improvements that have been achieved so far in the ABE fermentation in *Clostridia,* the maximum achievable yield of butanol in *Clostridia* is still considerably low and the reliability of the process is still insufficient for a butanol production process on an industrial scale.

Since *Clostridia* are sensitive to oxygen, *Clostridia*-fermentations need to be operated under strict anaerobic conditions, which makes it difficult to operate such fermentations on a large scale. Anaerobic fermentations generally result in low biomass concentrations due to the low ATP-gain under anaerobic conditions. In addition, Clostridia are sensitive to bacteriophages, causing lysis of the bacterial cells during fermentation. Since Clostridia fermentations are carried out at neutral pH, sterile conditions are essential to prevent contamination of the fermentation broth by eg. lactic acid bacteria, which lead to high costs for fermentations on an industrial scale (Zverlov et al. Appl. Microbiol. Biotechnol. Vol. 71, p. 587-597, 2006, Spivey, Process Biochemistry November 1978). Another disadvantage of butanol production in Clostridia is that undesirable by-products like, acetone, acetate and butyrate are also produced, which lowers the yield of butanol on carbon.

### Detailed description of the invention

In a first aspect, the present invention relates to the use of a solvent tolerant bacterium in a method for the fermentative production of a solvent, wherein the solvent is a four carbon alcohol. The solvent is preferably 1-butanol, 2-butanol, isobutanol, 2,3-butanediol or 2-butanone. More preferably, the solvent is produced in the fermentative production of 1-butanol.

The solvent tolerant bacterium used is a lactic acid bacterium, i.e. belonging to the *Lactobacillales* (see for instance www. nbci.nlm.nih.gov), preferably one which is food grade. In the context of the present invention, the phrase 'food grade' indicates that the bacterium may safely be used in food applications. Preferably, the solvent tolerant bacterium used is a lactic acid bacterium which is a member of the genera *Lactobacillus, Oenococcus* or *Leuconostoc.*

One advantage of members of the genera *Lactobacillus, Oenococcus* or *Leuconostoc* over other lactic acid bacteria is that *Lactobacilli, Oenococci and Leuconostoc* are well-established industrial organisms, which are widely used for industrial scale fermentations, in particular in the food industry, have GRAS status (generally recognized as safe) as fermentation agents in food and are not associated with health risks.

In a preferred embodiment, the solvent tolerant bacterium is a member of the genus *Lactobacillus.* More preferably, the solvent tolerant bacterium is a *Lactobacillus plantarum, a Lactobacillus casei, a Lactobacillus fructivorans, Lactobacillus homohiochii* or a *Lactobacillus helveticus* species or subspecies. Most preferably, the solvent tolerant bacterium used is a *Lactobacillus* as deposited under accession no. ATCC 8014, CBS 636.88, ATCC 15434, ATCC 27745, ATCC 10386, ATCC 8288, a *Lactobacillus* which is obtainable from Adriana Normandie, France as ADRIA 85L121 or a *Lactobacillus* which is obtainable from commercial rennet.

One advantage for *Lactobacilli* is that they may be used over a wide temperature range. They can be used both at temperatures as high as 40 or 45 degrees C and at lower temperatures. According to the present invention, the *Lactobacilli* are preferably used in a solvent production process which is carried out at a temperature lower than 37 degrees C. More preferably, the *Lactobacilli* are used at a temperature in the range of 35 to 20 degrees C or 32 to 20 degrees C. Even more preferably, the *Lactobacilli* are used at a temperature in the range of 30 to 20 degrees C or 28 to 20 degrees C. The ability to grow at lower temperatures is an advantage for solvent production processes, because solvent tolerance will be higher at lower temperature.

Yet another advantage is that *Lactobacilli* allow fermentation processes to be carried out at a wide range of pH values, i.e. pH 3-10. According to the present invention, the *Lactobacilli* are preferably used in a solvent production process which is carried out at a pH in the range of pH 3-7, more preferably at a pH in the range of pH 3-5.

Yet another advantage is that *Lactobacilli* are tolerant to high solvent concentrations. For example, they display growth rates of up to 0.2/hr at butanol concentrations in the range of 20 to 30 g/l butanol (2-3% w/v). They have IC₅₀ (%) values for butanol of at least 1.8 or 2.0 in liquid medium. Preferably, they have IC₅₀ (%) values for butanol of at least 2.2. or 2.4 in liquid medium. More preferably, they have IC₅₀ (%) values for butanol of at least 2.6, 2.8 or 3.0. Even more preferably, they have IC₅₀ (%) values for butanol of at least 3.2, .3.4, 3.6, 3.8 or 4.0 in liquid medium. In a preferred embodiment, *Lactobacilli* display growth rates of up to 0.2/hr at butanol concentrations in the range of 20 to 30 g/l butanol (2-3% w/v) and have an IC₅₀ (%) of at least 1.8 or 2.0, more preferably of at least 2.2. or 2.4, most preferably of at least 2.6, 2.8 or 3.0 when measured in a sealed space containing liquid medium, at 30 degrees C for maximally 15 hours, more preferably for maximally 10 hours. The container is preferably a microtiter plate.

Because of their versatility, i.e. their ability to grow at a wide range of temperatures and a wide range of pH values, and their high solvent tolerance, *Lactobacilli* are the ideal bacteria for use in solvent production processes. Preferably the *Lactobacilli* are used in a solvent production process in which the circumstances are unfavourable to other bacteria, because this will minimise the risk of bacterial contamination of the fermentation broth. In one embodiment, the *Lactobacilli* are used in a solvent production process wherein the pH is in the range of pH 3-5 and the temperature is in the range of 20 to 30 degrees C.

In another aspect, the present invention relates to a solvent tolerant lactic acid bacterium, wherein the lactic acid bacterium is tolerant to at least 2% w/v solvent and wherein the solvent is a four carbon alcohol. The solvent is preferably 1-butanol, 2-butanol, isobutanol, 2,3-butanediol or 2-butanone. The solvent tolerant bacterium is preferably a bacterium which has a IC₅₀ (%) value for butanol of at least 1.8 or 2.0 in liquid medium, at 30 degrees C for maximally 15 hours, more preferably for maximally 10 hours. Preferably, they have IC₅₀ (%) values for butanol of at least 2.2. or 2.4 in liquid medium. More preferably, they have IC₅₀ (%) values for butanol of at least 2.6, 2.8 or 3.0. Even more preferably, they have IC₅₀ (%) values for butanol of at least 3.2, .3.4, 3.6, 3.8 or 4.0 in liquid medium. Suitable examples of such bacteria include species from *Lactobacillus, Oenococcus* or *Leuconostoc.* Preferred embodiments of the first aspect are also applicable to the second aspect.

### EXAMPLES

### Example 1

### Bacterial strains

Lactobacilli strains were obtained from public sources (see Table 1) in frozen form and inoculated in de Man, Rogosa, Sharpe (MRS) medium.

**Table 1**

| | |
|---|---|
| *L. casei* | From commercial rennet |
| *L. casei* | ADRIA 85 L121 |
| *L. plantarum* | ATCC 8014 |
| *L plantarum* | CBS 636.88 |
| *L. homohiochii* | ATCC 15434 |
| *L. homohiochii* | ATCC 27745 |
| *L. helveticus* | ATCC 10386 |
| *L. fructivorans* | ATCC 8288 |

MRS medium oxoid (CM0359, Merck KgaA, Germany) was prepared according to manufacturer's instructions and filled out in 50 ml Schott flasks and sterilized at 121 degrees C during 15 minutes. 1 vial of thawed frozen culture was inoculated to 50 ml of this medium and the cultures were subsequently incubated overnight at 30 degrees C standing still or during 2 nights if no clear growth (whitening) had occurred yet. 20 microliter of this culture was inoculated to 180 microliter of microtiter plate (MTP) medium. The pH of this medium was approximately 6.0.

### Example 2

### Solvent evaporation experiments

Microtiter plates were sealed to minimise losses due to solvent evaporation. However, experiments (Table 2) showed that even in sealed microtiter plates losses of more than 10% may occur. Butanol evaporation is faster at higher temperatures, but will be less than 5% if incubated for 10 hours or less at 30 degrees C. After 96 hours at 37 degrees C, approximately 50% of the butanol was evaporated from sealed MTPs (not shown).

**Table 2**

| Concentration in medium | Concentration after 24 h incubation | fraction left after 24 h incubation | loss | predicted at 10 hrs |
|---|---|---|---|---|
| [g/L] | [g/L] | | [%/hr] | [g/L] |
| 0 | 0 | | | 0 |
| 5 | 4.7 | 0.94 | 0.0025 | 4.9 |
| 10 | 9.7 | 0.97 | 0.0015 | 9.9 |
| 15 | 13.4 | 0.89 | 0.0046 | 14.3 |
| 25 | 21.6 | 0.86 | 0.0058 | 23.6 |
| 40 | 33.9 | 0.85 | 0.0064 | 37.5 |

### Example 3

### Butanol tolerance of Lactobacilli.

Butanol tolerance of Lactobacilli was measured in microtiter plates (MTP). MTP medium for lactic acid bacteria was also based on MRS medium, but some water was eliminated in order to compensate for the butanol to be added. The media contained 0-5-10-15-25 and 40 g/L of butanol respectively prior to inoculation. After inoculation, every 20 minutes the optical density at 620 nm was measured in the wells of the microtiter plate using a Multiscan Ascent photometer (Thermo Fisher Scientific, Inc.), while the temperature was controlled at 30 degrees C. Total time of measurement was maximally 24 hours. Before measurements were taken, the microtiter plates were shaken for 30 seconds to homogenize any settled biomass.

For retrieving kinetic data, the incubation period was limited to 5-10 hours at 30 degree C to keep the error in butanol concentrations due to butanol evaporation as low as possible in the sealed microtiter plates (see Example 2 and Table 2).

Growth response of various Lactobacilli towards butanol in the medium is represented in Table 3. The growth rate was calculated using the exponential phase of the growth curve, based on the optical density measurement. The relative growth rate was calculated using the reference at 0 g/L as 1.00. The results show that Lactobacilli can still grow at a butanol concentration of 2.5 w/v%.

**Table 3 Relative growth rate**

| strain | *L. casei* from commercial rennet | *L. casei* ADRIA 85 L121 | *L. plantarum* ATCC 8014 | *L. fructivorans* ATCC 8288 |
|---|---|---|---|---|
| Butanol concentration [w/v%] | | | | |
| 0 | 1.00 | 1.00 | 1.00 | 1.00 |
| 0.5 | 0.88 | 0.96 | 0.97 | 0.94 |
| 1 | 0.79 | 0.89 | 0.90 | 0.89 |
| 1.5 | 0.68 | 0.73 | 0.77 | 0.78 |
| 2.5 | 0.24 | 0.21 | 0.37 | 0.28 |

IC₅₀(%) values were determined for the *Lactobacilli* at 30 degrees C by using a polynomial second order fit through the relative growth rate data and are presented in Table 4. All tested *Lactobacilli* have an IC₅₀(%) value of 1.9 or higher.

**Table 4**

| | IC₅₀% |
|---|---|
| | w/v % |
| *L. casei* from commercial rennet | 1.9 |
| *L. casei* ADRIA 85 L121 | 2.0 |
| *L. plantarum* ATCC 8014 | 2.2 |
| *L. fructivorans* ATCC 8288 | 2.1 |

## Claims

1. Use of a solvent tolerant lactic acid bacterium in a solvent production process, wherein the solvent is a four carbon alcohol.

2. Use according to claim 1, wherein the four carbon alcohol is 1-butanol, 2-butanol, isobutanol, 2,3-butanediol or 2-butanone.

3. Use according to claim 1 or 2, wherein the lactic acid bacterium belongs to the genus *Lactobacillus, Oenococcus* or *Leuconostoc.*

4. Use according to claim 3 wherein the bacterium is a *Lactobacillus* plantarum, a *Lactobacillus* casei or a *Lactobacillus* fructivorans.

5. Use according to claims 1-4, wherein the bacterium has a IC₅₀(%) value for butanol of at least 1.8.

6. A solvent tolerant bacterium, wherein the bacterium is tolerant to at least 2 w/v % of a four carbon alcohol solvent.

7. A solvent tolerant bacterium according to claim 6, wherein the bacterium has a IC₅₀ (%) of at least 1.8

8. A solvent tolerant bacterium according to claim 6 or 7, wherein the bacterium belongs to the genus *Lactobacillus, Oenococcus* or *Leuconostoc.*

9. A solvent tolerant bacterium according to claim 8, wherein the solvent is 1-butanol, 2-butanol, isobutanol, 2,3-butanediol or 2-butanone.
